# EUROPÄISCHE PATENTSCHRIFT

(11) **EP 2 352 426 B1**
(45) Veröffentlichungstag und Bekanntmachung des Hinweises auf die Patenterteilung: **01.02.2017**
(21) Anmeldenummer: 09751777.5
(22) Anmeldetag: 14.10.2009
(51) Int. Cl.: A63B 69/00, A63B 71/06, A63B 24/00, A61B 5/103, A63B 22/02, A63B 23/04

(54) **ANORDNUNG ZUM TRAINING DES GANGES**
ARRANGEMENT FOR GAIT TRAINING
DISPOSITIF POUR L'ENTRAÎNEMENT À LA MARCHE

(30) Priorität: 19.11.2008 DE 102008058020
(43) Veröffentlichungstag der Anmeldung: 10.08.2011
(73) Patentinhaber: Zebris Medical GmbH, 88316 Isny im Allgäu (DE)
(72) Erfinder: BRUNNER, Wolfgang, 88316 Isny (DE)
(74) Vertreter: Heinze, Ekkehard
(86) Internationale Anmeldenummer: PCT/EP2009/007379
(87) Internationale Veröffentlichungsnummer: WO 2010/057552

(56) Entgegenhaltungen:
- EP-A1- 1 386 642
- WO-A1-01/93960
- WO-A2-03/025615
- DE-U1-202005 010 319
- US-A1- 2003 211 896
- US-A1- 2004 192 511
- US-A1- 2006 247 104
- US-B1- 6 918 859

## Beschreibung

Die Erfindung betrifft eine Anordnung zum Training des Ganges.

Vorrichtungen zur Erfassung von Druck- bzw. Kraftverteilungen sind an sich bekannt, beispielsweise aus der DE 36 42 088 C2 und der DE 25 29 475 C3.

Viele der bekannten Vorrichtungen können als Plattformen zur biomechanischen Ganganalyse eingesetzt werden, bei denen der Gang eines Wirbeltiers, insbesondere eines Menschen, gegebenenfalls aber auch eines Pferdes oder Hundes etc., untersucht und analysiert wird. Hierbei besteht aber der Nachteil, dass immer nur ein einziger Schritt und ein einzelner Abrollvorgang aufgenommen werden kann. Um ein natürliches Gangverhalten zu erhalten, ist es dagegen notwendig, den Gang über eine längere Zeit aufzunehmen.

Es wurden daher auch bereits Vorrichtungen und Verfahren zur Ganganalyse, die sich eines Laufbandes bedienen, vorgeschlagen. Beispielhaft sei hierzu hingewiesen auf die DE4027317C1, US6010465A, oder auch die US2006/0247104.

Ferner wird in R. Kram und A.J. Powell: "A treadmill-mounted force platform" Appl. Physiol. 67 (4): 1692-1698 (1989) eine Messvorrichtung als bekannt beschrieben, bei der ein Laufband über eine Messplattform bzw. Messfläche gezogen wird und somit eine fortlaufende Erfassung von Kräften möglich wird.

Die erstere dieser Druckschriften beschreibt ein Laufband, welches aus einer Vielzahl von Gliedern aufgebaut ist, von denen jedes eine matrixförmige Anordnung von Druck- bzw. Kraftsensoren umfasst, während die zweite ein Laufband mit unter der Bandoberfläche liegender Messplatte mit einer matrixförmigen Anordnung von Druck- bzw. Kraftsensoren beschreibt. Beide Druckschriften lehren, dass mit der jeweiligen Sensorik eine Auswertungseinheit verbunden ist und die US 6,010,465 beschreibt den Aufbau und die Betriebsweise der Auswertungseinheit, etwa zur Auswertung der Position und eines zugeordneten Kraftwertes beim Auftreten auf das Laufband, beispielsweise zur Bestimmung von Drehmomenten und Lasten auf die Gelenke sowie bestimmten Gangparametern, relativ detailliert.

Mit Verbesserungen dieser bekannten Lösungen hinsichtlich der Ableitung differenzierter medizinischer bzw. sportphysiologischer Aussagen befasst sich die WO2007/131542A1 der Anmelderin. Diese lehrt insbesondere Mittel und Vorgehensweisen zur genauen und differenzierten Erfassung der tatsächlichen Geschwindigkeit des Laufbandes unter Nutzung der Zeit- und Ortsabhängigkeit von auf diesem beim Gehen oder Laufen eines Probanden aufgenommenen Druckverteilungsbildern.

Bekannt ist auch der Einsatz von Anzeigevorrichtungen, wie Anzeigeschirmen, bei Laufband-Anordnungen.

Aus der EP 1 145 682 A2 sind ein auf der Laufband-Technik aufbauendes Rehabilitationsgerät und -verfahren bekannt, bei dem eine Anpassung der Funktion des Laufbandes an den aktuellen Stand der wiederherzustellenden Geh- bzw. Lauffähigkeit eines Patienten vorgesehen ist. Insbesondere wird die Laufbandgeschwindigkeit an einen persönlichen Schrittzyklus des Nutzers angepasst, und das Gerät soll diesem zugleich ein Feedback geben. In einer speziellen Ausführung ist auch die Erfassung von beim Auftreten ausgeübten Druckkräften und deren Auswertung im Rahmen des Gesamtprogramms vorgesehen. Die Druckschrift beschreibt auch den Einsatz eines Anzeigeschirmes in Verbindung mit einer Eingabetastatur zur Anzeige der auf dem Laufband erzeugten Fußabdrücke und zur Einstellung von Laufbandparametern anhand dieser Anzeige.

Aus der US 6,231,527 B1 ist ebenfalls der Einsatz eines Anzeigeschirmes bei einer Laufbandvorrichtung für den sportmedizinischen bzw. Rehabilitations-Einsatz bekannt, wobei hier die Bilder verschiedener Kameras dargestellt werden können, die den Sportler/Patienten in seinen Bewegungen auf dem Laufband aufnehmen.

Auch die EP2060229A1 der Anmelderin beschreibt ein Laufbandsystem für den sportmedizinischen bzw. Rehabilitationseinsatz mit einer Bildwiedergabetechnik, bei dem außerdem Druckverteilungsmuster, die von einer auf dem Laufband laufenden Person erzeugt werden, erfasst und synchron zu dargestellten Bildern ausgewertet werden können.

Der Erfindung liegt die Aufgabe der Bereitstellung einer weiter verbesserten Vorrichtung der letztgenannten Art zugrunde, die sich besonders für sportmedizinische oder Rehabilitations-Zwecke eignet. Ein Ziel ist es, das System zu einem flexibelnutzbaren Trainings-/Therapiegerät weiter zu entwickeln und dabei die Nutzerakzeptanz weiter zu erhöhen.

Diese Aufgabe wird durch eine Anordnung mit den Merkmalen des Anspruchs 1 gelöst. Zweckmäßige Fortbildungen des Erfindungsgedankens sind jeweils Gegenstand der abhängigen Ansprüche.

Die Erfindung schließt, in Weiterentwicklung der oben erwähnten unveröffentlichten EP-Anmeldung, den Gedanken der Darstellung eines im Rehabilitations- bzw. Trainings-Zusammenhang stehenden Bildes direkt im Bereich der Lauffläche, also auf der oberen Oberfläche des Laufbandes bzw. bei Verwendung eines durchsichtigen Materials für das Laufband auch auf einer Anzeigefläche unterhalb des oberen Abschnitts des Laufbandes, ein. Sie schließt weiter den Gedanken ein, hier insbesondere Bilder oder Bildelemente darzustellen, die der Trainierende mit seinen Füßen treffen oder - im Gegenteil - nicht berühren soll und die von geeigneten Bildsignal-Erzeugungsmitteln unter angemessener Steuerung des Anzeige-Ablaufes bereitgestellt werden. Schließlich gehört zur Erfindung der Gedanke, die Auswertung der von den Füßen des Trainierenden stammenden Druckverteilungsmuster mit der Bildanzeige im Bereich des Laufbandes zu synchronisieren bzw. eine sonstige Anpassung dahingehend vorzunehmen, dass eine Auswertung des Ganges bzw. Laufes als direkte Reaktion des Trainierenden auf ihm angezeigte Bilder möglich wird.

Die erfindungswesentliche Bildanzeige im Bereich des Laufbandes kann auf verschiedene Weise und mit jeweils bekannten Anzeigevorrichtungen geschehen. In einer ersten Ausführung weisen die Anzeigemittel einen Bildprojektor zur Projektion von Bildern auch die Lauffläche des Laufbandes auf, insbesondere einen Laserbeamer, der an einen Kleinrechner (PC, Laptop, PDA...) als Bild- bzw. Videoquelle angeschlossen ist. Die projizierten Bilder müssen natürlich ggf. entzerrt werden, da u.U. der Projektor nicht direkt von oben sondern beispielsweise von schräg vorne angebracht werden sollte, um eine Verdeckung des projizierten Bildes durch die Füße zu vermeiden. Um die Laufumgebung möglichst natürlich zu gestallten, kann im Übrigen die Projektionsfläche vorn etwas nach oben gezogen sein, damit der Kopf des Patienten nicht allzu sehr nach unten geneigt werden muss.

Eine hierzu alternative Ausführung sieht vor, dass die Bildanzeigefläche durch mindestens einen Anzeigeschirm einer elektrooptischen Anzeigevorrichtung, insbesondere von LCD-, Plasma- oder OLED-Typ gebildet ist. Dies setzt selbstverständlich eine Ausführung des Laufbands aus durchsichtigem Material voraus, so dass die Bildanzeigefläche unterhalb seines (in Gebrauchsstellung) oben verlaufenden Abschnittes statisch angeordnet sein kann. Diese Ausführung aus (mindestens bereichsweise) durchsichtigem Material ist aber nicht notwendigerweise mit der letztgenannten Ausführung der optischen Anzeigemittel verbunden, sondern sie kann unter gewissen Umständen auch in Kombination mit einer Projektionstechnik sinnvoll sein.

Mit den Anzeigemitteln werden beispielsweise Schritte in Form von Kreisflächen oder in Form von Fußabdrücken auf den Laufgurt projiziert. Der Proband bzw Trainierende wird aufgefordert, seine Füße auf die projizierten Flächen zu setzten. Damit können die Patienten angehalten werden, bestimmte Schrittweiten oder Schrittbreiten einzuhalten. Werden anstatt von Kreisflächen Fußabdrücke aufprojiziert, so kann auch der Aufsetzwinkel der Füße vorgegeben werden. Die Drucksensormatrix ist mit den projizierten Flächen räumlich kalibriert, sodass direkt überprüft werden kann, ob die Füße tatsächlich richtig aufgesetzt wurden. Ggf. kann über ein Feedback-Signal der Patient aufgefordert werden, seine Schritte den Vorgaben anzupassen. Ebenso kann eine Therapie bzw. Erfolgskontrolle durchgeführt werden.

Abweichend von vorgegebenen Flächen zum Aufsetzen der Füße können natürlich auch Hindernisse projiziert werden, die nicht betreten werden sollen, sodass der Patient die verbotenen Flächen überspringen oder übersteigen muss und somit seine Koordination trainieren kann. Dies können z.B. Gegenstände oder Pfützen sein, bei deren Betreten ebenfalls ein Feedback-Signal ausgelöst wird oder ein Punkteabzug erfolgen kann.

In einer weiteren Ausführung umfasst die Anordnung zusätzliche optische Anzeigemittel zur Darstellung von Bildern auf einer weiteren Bildanzeigefläche, außerhalb der Lauffläche und insbesondere in Augenhöhe des Trainierenden, wobei die zusätzlichen Anzeigemittel ebenfalls mit den Bildsignal-Erzeugungsmitteln und der Anzeige-Ablaufsteuerung verbunden sind und insbesondere zu einer zusätzlichen Visualisierung der Lauffläche und/oder Laufumgebung genutzt werden. Auf diesem Bildschirm werden vorzugsweise zusätzlich die auf dem Laufband projizierten Muster dargestellt. Diese werden nun mit den virtuellen Fußabdrücken der Sensormatrix überlagert. Der Patient hat somit die Wahl, seine eigenen Füße auf dem Band oder die virtuellen Füße oder Fußabdrücke auf dem Bildschirm zu betrachten und in die richtige Position zu bringen.

In einer weiteren Ausführung ist es vorgesehen, in die oder unter der Lauffläche entweder feste Erhebungen bzw. Vertiefungen oder Aktoren einzubauen mit denen sich auf der Lauffläche Erhöhungen oder insbesondere auch lokal als nachgiebig bzw. elastisch oder aber besonders hart empfundene Bereiche der Lauffläche realisieren lassen. Dies könnte z.B. mit einer Matrix aus luft- oder flüssigkeitsgefüllten Kammern realisiert werden. Diese werden, wie die Komponenten zur Auswertung der Druckverteilungsbilder und zur Synchronisation mit den Anzeige-Bildern, von einer gemeinsamen Steuereinheit gesteuert und können für Provokationstests verwendet werden.

Bei der ersten Variante ist es insbesondere zweckmäßig, wenn das Endlosband mit gang-wirksam strukturierter Oberfläche eine Positionskodierung zur Positionszuordnung von Oberflächenelementen aufweist, die Verarbeitungseinheit Profilspeichermittel zur Speicherung des Profils der gang-wirksam strukturierten Oberfläche aufweist und ihr ein Positionssignalempfänger zur Adressierung des Profilspeichers zugeordnet ist und in der Verarbeitungseinheit ein Verarbeitungsalgorithmus implementiert ist, mit dem die Druckverteilungsbilder den Oberflächenelementen der Lauffläche zugeordnet werden.

Eine vorteilhafte Ausführung der zweiten Variante zeichnet sich dadurch aus, dass der Anordnung von Aktoren eine Profilsteuereinheit zugeordnet ist, die Steuersignale zur Betätigung der Aktoren zur Ausbildung eines vorbestimmten dynamischen Profils (herkömmlichen Oberflächenprofils und/oder Elastizitäts- bzw. Härteprofils) der Lauffläche ausgibt, die Verarbeitungseinheit einen Steuersignalempfänger zum Empfang der durch die Profilsteuereinheit ausgegebenen Steuersignale als Positionszuordnungssignale aufweist und in der Verarbeitungseinheit ein Verarbeitungsalgorithmus implementiert ist, mit dem die Druckverteilungsbilder dem dynamischen Profil der Lauffläche zugeordnet werden. Es versteht sich, dass sinnvollerweise zu lokalen Bereichen mit differierender Elastizität bzw. Härte Bildelemente projiziert werden, die diese Differenzierung für den Trainierenden (Probanden) auch visuell erlebbar machen.

In Kombination hiermit oder auch als Alternative hierzu ist eine Ausführung vorgesehen, bei der das Laufband insgesamt auf Aktoren zur Erzeugung einer globalen Nachgiebigkeit bzw. Elastizität der gesamten Lauffläche gelagert ist. Statt einer Simulation lokaler Defekte des Untergrundes gelingt mit dieser Ausführung die Simulation einer großflächigen Elastizität bzw. Nachgiebigkeit des Untergrundes.

Weitere sinnvolle Ausgestaltungen der Erfindung betreffen die Bereitstellung der Bildsignale. So ist in einer Ausführung vorgesehen, dass die Bildsignal-Erzeugungsmittel eine Bildelement-Speichereinheit zum Speichern einer Vielzahl vordefinierter Bildelemente und wahlweise einen Bildelement-Mischer zum Mischen von aus der Speichereinheit abgerufenen Bildelementen nach einer Vielzahl vorbestimmter Mischprogramme aufweist. Besonders vorteilhaft kann zusätzlich vorgesehen sein, dass die Bildsignal-Erzeugungsmittel einen Videospeicher zur Speicherung von Bildfolgen und wahlweise einen Video-Bildelement-Mischer zum Mischen von aus dem Videospeicher ausgelesen Bildfolgen, wahlweise mit aus der Bildelement-Speichereinheit ausgelesenen Bildelementen, aufweist. Mit diesen Mitteln lässt sich sehr realitätsnah ein natürlicher Untergrund optisch (und bei Hinzunahme entsprechender Aktoren, wie weiter oben erwähnt) auch taktil simulieren, und in diesen Untergrund können Bilder bzw. Bildelemente eingeblendet sein, die dem Trainierenden bestimmte Anforderungen auferlegen und somit ein besonders effizientes Training ermöglichen.

Eine durch die Anordnung bereitgestellte Benutzerführung, mit optischer und/oder akustischer Ausgabe von anzeigebegleitenden Handlungsanweisungen, dient zur Realisierung von Trainings- bzw. Rehabilitationsprogrammen. Das kann sich wie folgt darstellen: Der Patient läuft zunächst in seinem gewohnten Laufstil. Mit Hilfe der im Laufband integrierten Drucksensormatrix werden die charakteristischen Gangparameter, insbesondere der Schrittlänge,. Spurbreite, Fußrotationswinkel oder aufgebrachten Kraftwerte, erfasst. Diese Parameter können dann als Ausgangswerte für das Training verwendet werden. Die auf das Laufband projizierten Muster werden, ausgehend von dieser Einstellung, innerhalb eines Trainingsablaufs automatisch auf die vom Therapeuten gewünschte Zielgröße geändert. Die Änderung der Werte kann linear, exponentiell oder mit jeder anderen mathematischen Funktion durchgeführt werden. Danach kann eine automatische Analyse mit einem Soll-Ist-Vergleich durchgeführt werden, d.h. es wird überprüft, wie gut der Patient die Vorgaben einhalten konnte. Soll vom Patienten die aufgebrachte Kraft in Teilbereichen des Fußes oder des ganzen Fußes verändert werden, so wird er durch ein akustisches Signal oder eine Sprachausgabe instruiert. Damit erfolgt z.B. die Anweisung, einen Fuß stärker oder weniger stark zu belasten.

In einer besonderen Ausführung können vorgegebene Schrittweiten oder Breiten im Laufe eines Trainingsdurchgangs stetig verändert (z.B. vergrößert) werden, um den Patienten langsam an die Trainingsziele heranzuführen. Dem dient eine Ausführung, bei der die Anzeige-Ablaufsteuerung einen Programmspeicher zur Speicherung einer Vielzahl vorbestimmter Anzeige-Abläufe und/oder Geschwindigkeitsund/oder Relativpositions-Einstellmittel zur Geschwindigkeitseinstellung eines Anzeige-Ablaufes bzw. zur Vorgabe von Relativpositionen zwischen Bildelementen einer Anzeige aufweist. Auch ist es möglich, per Zufallsgenerator die Symbole zum Setzen der Schritte permanent zu verändern, um die Koordination des Patienten zu trainieren und um eingeschliffene Gangmuster zu verlassen und damit den Therapieerfolg zu verbessern. Im Übrigen kann der Zufallsgenerator neben Schritt-Aufsetzmustern auch sonstige Bildelemente (etwa Hindernisse) in stochastisch variierter Form präsentieren und für den Trainierenden damit die Koordinations-Anforderungen weiter steigern.

Aus den vorstehenden Ausführungen ergibt sich bereits, dass die vorgeschlagene Anordnung zweckmäßigerweise mit einer Benutzerführungseinheit zur optischen und/oder akustischen Ausgabe von anzeigebegleitenden Handlungsanweisungen, insbesondere über Ohrhörer und/oder Texteinblendungen im Bereich der Lauffläche ausgerüstet ist.

Vorteile und Zweckmäßigkeiten der Erfindung ergeben sich um Übrigen aus der nachfolgenden Beschreibung bevorzugter Ausführungsbeispiele anhand der Figuren. Von diesen zeigen:
Fig. 1 eine schematische Darstellung einer ersten Ausführungsform der Erfindung,
Fig. 2 eine schematische Darstellung einer zweiten Ausführungsform der Erfindung,
Fig. 3 eine Detailansicht zu einer weiteren Ausführungsform,
Fig. 4 eine Detailansicht zu einer weiteren Ausführungsform und
Fig. 5 eine bildliche Darstellung, wie sie im Rahmen eines Trainingsprogramms auf der Lauffläche der Anordnung gegeben werden kann.

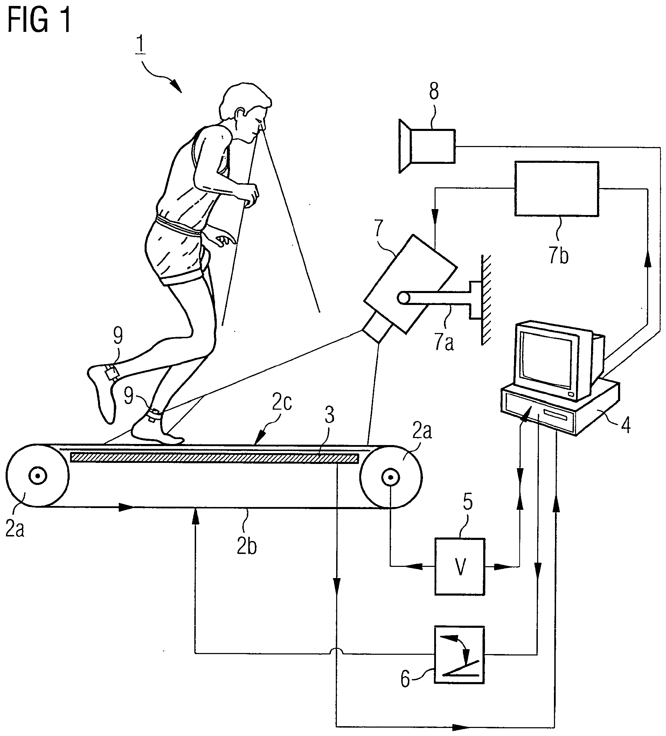

Fig. 1 zeigt ein Laufband-Trainingssystem 1, welches ein über zwei Walzen 2a laufendes Laufband 2b umfasst, unter dessen oberer, vom Benutzer als Lauffläche 2c genutzten Oberfläche eine räumlich hochauflösende Druckerfassungsplatte 3 mit einer Vielzahl von (nicht einzeln bezeichneten) matrixförmig angeordneten Drucksensoren zur Erfassung von durch den Benutzer beim Auftreten auf das Laufband erzeugten Druckerfassungsbildern vorgesehen ist. Die eine der beiden Laufrollen 2a ist angetrieben und zieht das Band 2b mit einer vorbestimmten Geschwindigkeit, die über eine Verarbeitungs- und Steuereinheit 4 der Anordnung und einen Geschwindigkeitsregler 5 eingestellt wird. Des weiteren kann (was in der Figur lediglich symbolisch dargestellt ist) über ein geeignetes Neigungs-Stellglied 6, welches ebenfalls Störsignale von der Verarbeitungs- und Steuereinheit 4 empfangen kann, eine Neigung des gesamten Laufbandes bedarfsgerecht eingestellt oder wahlweise auch nur sein vorderer Teil etwas aufgerichtet werden werden.

Bei der in Fig. 1 dargestellten, stark vereinfachten Ausführung gelangen den eingestellten Geschwindigkeitswert des Laufbandes charakterisierende Signale vom Geschwindigkeitsregler 5 zurück zur Verarbeitungs- und Steuereinheit 4 und dienen dort zur Synchronisation einer bildlichen Darstellung auf der Lauffläche 2c mittels eines Projektors (Laserbeamers) 7, die aus vorgespeicherten Bildelementen und/oder Bildfolgen generiert wird (vgl. dazu weiter unten).

Die bildliche Darstellung wird anhand der Geschwindigkeitssignale derart gesteuert, dass - insbesondere in Verbindung mit einer weiter unten beschriebenen speziellen Ausgestaltung - dem Nutzer eine insgesamt stimmige Simulation einer Laufumgebung, vorteilhaft verknüpft mit der Einblendung von mit den Füßen zu treffenden Markierungen und/oder mit der Simulation von zu überwindenden oder zu vermeidenden Hindernissen, präsentiert wird. Abweichend von der Darstellung in der Figur, kann auch die tatsächliche Geschwindigkeit des Laufbandes über eine geeignete (nicht dargestellte) Sensorik erfasst und der Messwert der Verarbeitungs- und Steuereinheit 4 zum Zweck der (gewissermaßen rückgekoppelten) Ablaufsteuerung der Bilddarstellung und synchronisierten Auswertung der Druckverteilungsmuster zugeführt werden.

In der Figur ist dargestellt, dass der Projektor 7 an einer Wandhalterung 7a winkelverstellbar befestigt ist, so dass die Projektionsrichtung mit der Ebene des Laufbandes einen variablen Winkel einschließt. Um Verzerrungen der aus der Verarbeitungs- und Steuereinheit 4 bereitgestellten Bilder bzw. Bildelemente durch den spitzen Projektionswinkel zu vermeiden, ist dem Projektor 7 ein Bildsignal-Entzerrer 7b vorgeschaltet. Dieser kann in Abhängigkeit von der tatsächlichen Winkelstellung des Projektors 7 in der Halterung 7a arbeiten, das ist aber in der Figur aus Gründen der Übersichtlichkeit nicht dargestellt. Im Übrigen ist zur Vervollständigung der Benutzerschnittstelle eine (hier als Lautsprecher symbolisierte) Audiostufe 8 vorgesehen, über die der Trainierende zusätzliche akustische Trainingsanweisungen erhalten kann. Die Audiostufe 8 kann beispielsweise auch als Headset bidirektional ausgeführt sein, so dass der Trainierende außerdem akustisches Feedback (etwa eine Bestätigung erhaltener Anweisungen oder Antworten auf Ihm gestellte Fragen) liefern kann.

Zur Durchführung von Trainingsaufgaben am Laufband-System kann es von Interesse sein, die Höhe des Abhebens der Füße vom Band zu erkennen z.B. dann, wenn der Proband ein virtuelles Hindernis übersteigen soll. Deshalb hat der Proband in einer weiteren Ausführungsform jeweils einen Sensor 9 an den Füßen befestigt, dessen Signale mittels einer an sich bekannten (hier nicht dargestellten) Positionserfassungs-Sensorik erfasst werden können, um Rückschlüsse über die Lage bzw. die Höhe der Füße zu geben. Die Sensoren arbeiten vorzugsweise zeitsynchron mit den Sensoren der Druckverteilungsmatrix. Eine genaue Zeitsynchronisierung kann ggf. über ein Infrarot- oder Funksignal oder über eine Detektion des Zeitpunktes des Auftretens hergestellt werden.

Die Sensoren 9 können als Beschleunigungssensoren oder mehrachsigen Beschleunigungssensoren ausgeführt sein und sind ggf. über Funk mit dem Auswerterechner 4 verbunden. Aus den Beschleunigungssignalen kann die Position der Füße errechnet werden, insbesondere dann, wenn zusätzlich die Zeit- und Ortsabhängigkeit der Druckverteilungsmuster in die Berechnung mit eingehen können. In erweiterten Anordnungen können Inertialsensorsysteme zur Anwendung kommen, bei denen zusätzlich Gyroskope oder Sensoren zur Detektion des Erdmagnetfeldes eingesetzt werden. Solche Sensoren können natürlich auch an anderen Körperabschnitt befestigt werden, so dass die Bewegung der kompletten unteren Extremitäten oder des ganzen Körpers gemessen und dargestellt werden kann. Die Sensoren 9 können aber auch nach anderen Messprinzipien arbeiten, zum Beispiel auf der Basis aktiver oder passiver Lichtmarker die von stationären Kameras aufgenommen werden, Magnetfeldesensoren oder Sensoren die Ultraschallwellen zu stationären Empfängern abgeben oder von dort empfangen und aus der Laufzeit des Schalls die Position der Füße bestimmen.

Die Drucksensoren der Druckerfassungsplatte können wahlweise eine analoge oder - in einer vereinfachten und kostengünstigeren Ausführung - eine digitale Ansprechcharakteristik (Aus/Ein-Charakteristik) haben. Beide Varianten haben für bestimmte Anwendungen ihre Berechtigung, und der Auswahl einer der Varianten wird der Systementwerfer nach den primären Einsatzanforderungen treffen.

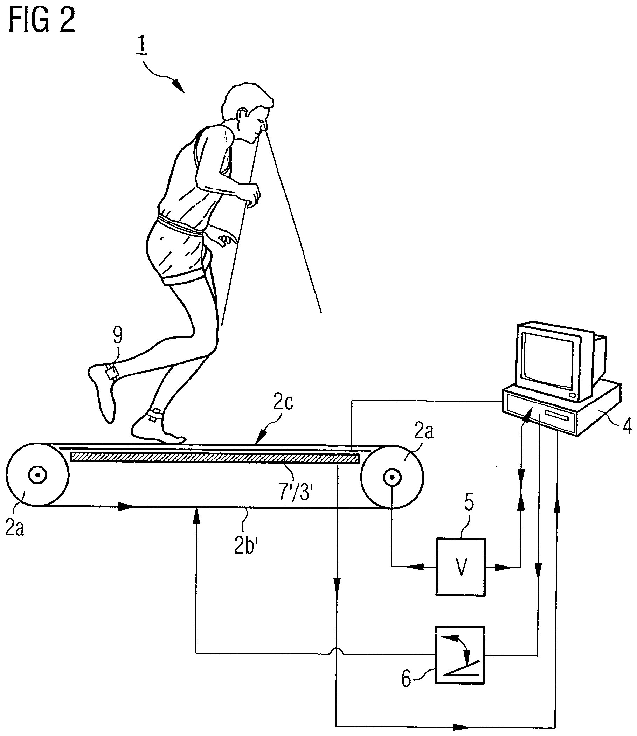

Fig. 2 zeigt eine Abwandlung der in Fig. 1 dargestellten und weiter oben beschriebenen Anordnung. Insoweit hier die gleichen Komponenten wie bei jener Anordnung eingesetzt werden, sind diese mit den gleichen Bezugsziffern wie in Fig. 1 bezeichnet und werden hier nicht nochmals erläutert.

Die wesentliche Abwandlung besteht im Einsatz eines großflächigen elektrooptischen Touchscreen 7' anstelle eines Projektors als Anzeigeeinrichtung. Die Oberfläche des Touchscreen bildet eine unterhalb des in Gebrauchslage oberen Abschnittes des Endlosbandes 2b liegende Anzeigefläche 2c', und zugleich bildet der Touchscreen eine neuartige Druckerfassungsplatte 3'. In Abwandlung des Touchscreen-Prinzips und mit Blick auf die Gestehungskosten der Anordnung kann diese kombinierte Anzeige-/Druckerfassungsvorrichtung durch eine matrixartige, alternierende Anordnung aus mehreren kleineren elektrooptischen Anzeigeelementen (etwa LCD-Displays) und jeweils benachbarten kleineren Druckerfassungsplatten ersetzt sein, oder es kann ein flexibler und druckunempfindlicher Anzeigeschirm (beispielsweise vom OLED-Typ) über eine normale Druckerfassungsplatte gelegt sein.

In allen Fällen ist das Endlosband 2b zumindest im mittleren Bereich seiner seitlichen Erstreckung aus einem durchsichtigen Material zu bilden, um dem Trainierenden eine Wahrnehmung der auf der Anzeigefläche 2c' dargestellten Bilder zu ermöglichen.

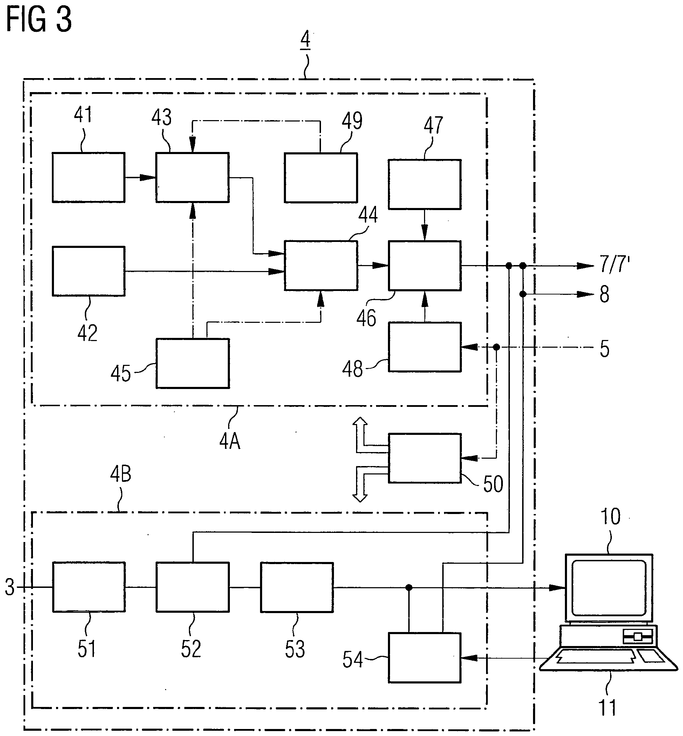

Fig. 3 zeigt wesentliche Komponenten der Verarbeitungs- und Steuereinheit 4 der in Fig. 1 oder 2 gezeigten Anordnung in einer Detaildarstellung. Ausgeklammert ist hier der in Fig. 1 separat dargestellte Bildsignal-Entzerrer, der auch nur bei einer Ausführung der Anordnung mit schräg auf das Laufband gerichtetem Projektor zum Einsatz kommt.

In einem Anzeigesteuerteil 4A umfasst die Verarbeitungs- und Steuereinheit 4 eine Bildelement-Speichereinheit 41 und einen Videospeicher 42, denen ein Bildelement-Mischer 43 und schließlich ein Video-Bildelement-Mischer 44 zur Generierung von Bildfolgen mit vorbestimmten Bildelement-Einblendungen nachgeschaltet sind. Symbolisch ist zudem dargestellt, dass beide Mischer 43, 44 des Weiteren von Steuersignalen eines Zufallsgenerators 45 beeinflussbar sind. Dem zweiten Mischer 44 ist zudem eine Anzeige-Ablaufssteuerung 46 nachgeschaltet, welcher ein Ablaufprogrammspeicher 47 und ein Geschwindigkeitsregler 48 zugeordnet sind. Eine Bildelement-Positionssteuerung 49 ist steuersignalmäßg mit dem Bildelement-Mischer 43 verbunden und wirkt auf diesen ein, um Relativpositionen von Bildelementen in der letztendlichen Darstellung zu variieren. Der Geschwindigkeitsregler 48 kann durch Signale des (in dieser Figur nicht dargestellten) Geschwindigkeitsreglers 5 des Laufbandes beeinflusst werden.

Zugleich werden diese Signale einer Systemsteuereinheit 50 der Anordnung zugeführt, die die verschiedenen Steuervorgänge der Anzeige- und Auswertungsfunktionen synchronisiert und erforderliche Anpassungen der Datenströme und -formate ausführt. Das ist in der Figur durch in auf den Anzeige-Steuerteil 4A und den Auswertungsabschnitt 4B gerichtete Doppelpfeile symbolisiert.

Zum Auswertungsabschnitt 4B gelangt zudem das am Ausgang der Anzeige-Ablaufsteuerung bereitgestellte endgültige Bildsignal und andererseits das (raumzeitlich aufgelöste) Ausgangssignal der Druckverteilungsplatte 3. Letzteres wird in einer Drucksignal-Vorverarbeitungsstufe 51 von Störsignalen und Artefakten befreit, in einer Drucksignal-Zeitanpassungsstufe 52 in zeitlicher Synchronität und in einer Drucksignal-Positionsanpassungsstufe 53 in räumliche Synchronität zu den Bildsignalen gebracht und in einer Trainings-Auswertungsstufe (Haupt-Verarbeitungsstufe) 53 anhand eines vorbestimmten Trainingsauswertungsprogramms verarbeitet, und die Ergebnisse werden auf einer separaten Anzeigeeinheit 10 des Therapeuten ausgegeben. Sie können zudem - zusammen mit über eine Eingabeeinheit 11 des Therapeuten eingegebenen Instruktionen - in einer Benutzerführungsstufe 54 zu Anweisungen an den Trainierenden verarbeitet werden, die über die diesem zugeordneten Anzeigeeinheit 7 bzw. 7' und wahlweise die Audiostufe 8 ausgegeben werden.

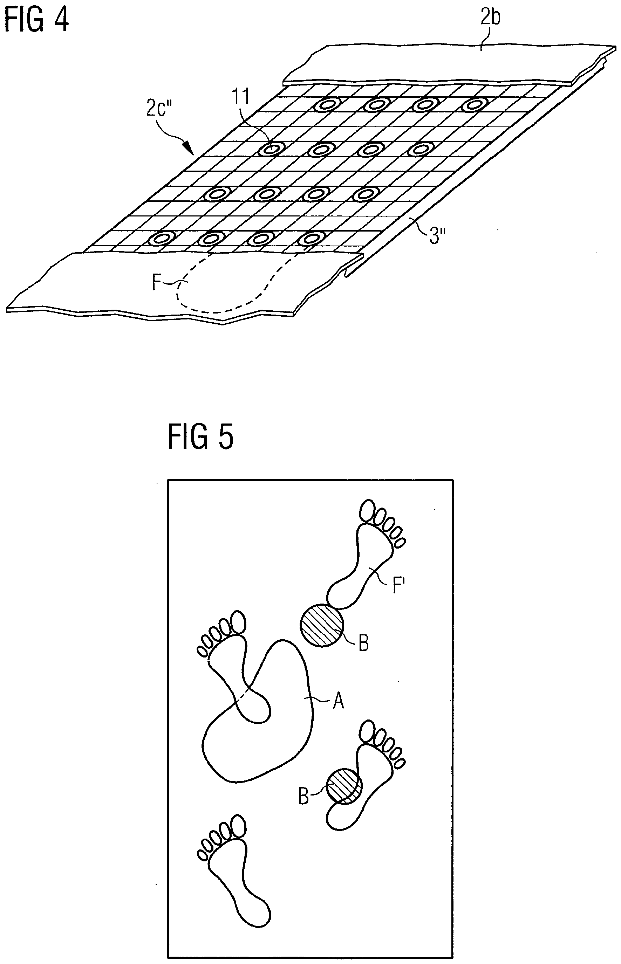

Fig. 4 zeigt in einer skizzenhaften perspektivischen Darstellung (teilweise geschnitten) einen Abschnitt der Lauffläche bei einer Ausgestaltung der erfindungsgemäßen Anordnung, in der in eine modifizierte Druckerfassungsplatte 3" eine regelmäßige Anordnung von Aktoren 12 eingebaut ist, die einzeln ansteuerbar sind und durch deren geeignete Ansteuerung ein härterer oder weicherer oder lokal einen bestimmten Grad an Elastizität aufweisender Untergrund simuliert werden kann. Auf dem Endlosband 2b ist die Kontur einer Ferse eines Fußes f gezeigt, die auf das Band projiziert wird und dem Trainierenden die Anweisung verdeutlichen soll, seinen Fuß auf jene Stelle zu setzen. Die Aktoren sind hier lediglich schematisch dargestellt; in der praktischen Ausführung können es beispielsweise mechanische Stößel oder gesteuert befüllbare Luft- oder Flüssigkeitskammern sein. Auch die matrixartige Anordnung einzelner Aktoren in Fig. 4 ist lediglich beispielhaft zu verstehen; sie können stattdessen auch in kleineren Gruppen, in Zeilen oder in Spalten angeordnet sein. Wichtig ist in jedem Fall, dass in der Umgebung der Aktoren eine ausreichende Anzahl von Drucksensoren angeordnet ist, um damit ein aussagekräftiges Druckverteilungsbild des seinen Fuß dort setzenden Probanden zu gewinnen.

Weiterhin versteht sich, dass die Steuerung der Aktoren über die Verarbeitungs- und Steuereinheit der Anordnung in einer Weise erfolgt, dass sie einem bestimmten Trainingsprogramm entspricht und gegebenenfalls von einer Benutzerführung begleitet und andererseits auf eine Bilddarstellung auf der Lauffläche (etwa die Darstellung eines Steinchens oder einer Pfütze etc.) abgestimmt und verlässlich mit dieser synchronisiert ist. Entsprechende Ergänzungen der in Fig. 3 gegebenen und oben erläuterten Darstellung der Verarbeitungs- und Steuereinheit 4 liegen im Rahmen fachmännischen Handelns und werden daher hier nicht genauer erläutert. Fig. 5 zeigt als Beispiel für eine dem Trainierenden bereitzustellende bildliche Darstellung auf der Lauffläche (vereinfacht) eine Serie von Fußabdrücken f', kombiniert mit Untergrund-Merkmalen A (Pfütze bzw. "weiche" Stelle) und B (Stein bzw. harte Erhebung). Aus den obigen Ausführungen ergibt sich, dass der Eindruck dieser Hindernisse nicht nur durch eine visuelle, sondern in Kombination hiermit auch durch eine taktile Wahrnehmung erweckt wird, die mit Hilfe einer Anordnung von Aktoren der Lauffläche der in Fig. 4 gezeigten Art realisiert wird.

## Patentansprüche

1. Anordnung (1) zum Training des Ganges, mit
einem über mindestes zwei Laufrollen (2a) geführten und als Laufband dienenden Endlosband (2b), dessen eine Oberfläche als Lauffläche (2c; 2c') dient,
einer Sensorik zur Bestimmung einer Druck-/Kraftverteilung auf einer unterhalb der Lauffläche befindlichen Kraftmessplatte (3; 3'), die auf der dem Endlosband zugewandten Seite eine Vielzahl von Druck-/Kraftsensoren aufweist,
einer mit den Druck-/Kraftsensoren eingangsseitig verbundenen Auswertungseinheit (4), welche Druckverteilungen detektiert, die durch einen gehenden oder laufenden Probanden auf der Lauffläche erzeugt werden,
einer eingangsseitig mit der Auswertungseinheit verbundenen Verarbeitungseinheit (51 - 53), welche aus den Druckverteilungen Werte bzw. Signale erzeugt, die den Gang des Probanden charakterisieren,
optischen Anzeigemitteln (7; 7') zur Darstellung von Bildern im Bereich der Lauffläche, die mit Bildsignal-Erzeugungsmitteln (41 - 45) und einer Anzeige-Ablaufsteuerung (4b) zur Erzeugung und zeitlich-räumlichen Positionssteuerung (49) von Bildern bzw. Bildelementen verbunden sind, sowie
einer Synchronisations- und Anpassungsstufe (7b) zur Synchronisation und Anpassung der Verarbeitung der Druckverteilungsbilder mit der bzw. an die Bilddarstellung im Bereich der Lauffläche,
**dadurch gekennzeichnet, dass**
die Anzeigemittel einen Bildprojektor (7) zur Projektion von Bildern auf die Lauffläche des Laufbandes aufweisen oder das Endlosband mindestens in wesentlichen Abschnitten aus durchsichtigem Material gebildet und eine Bildanzeigefläche unterhalb seines in Gebrauchsstellung oberen Abschnittes vorgesehen ist, und die Bildanzeigefläche durch mindestens einen Anzeigeschirm einer elektrooptischen Anzeigevorrichtung gebildet ist.

2. Anordnung nach Anspruch 1, wobei der Projektor (7) in Gebrauchsstellung der Anordnung vor oder im vorderen Bereich und/oder seitlich oberhalb desselben angeordnet ist derart, dass die Projektionsrichtung mit der Ebene der Lauffläche einen spitzen Winkel einschließt.

3. Anordnung nach Anspruch 2, wobei der Projektor (7) in einer winkelverstellbaren Halterung (7a) zur Einstellung einer Projektionsrichtung aufgenommen und ihm ein Bildsignal-Entzerrer (7b) vorgeschaltet ist, welcher eine Entzerrung von durch die Bildsignal-Erzeugungsmittel gelieferten Bildern bzw. Bildelementen in Abhängigkeit vom eingestellten Winkel zwischen Projektionsrichtung und Lauffläche bewirkt.

4. Anordnung nach einen der vorangehenden Ansprüche, wobei die Bildanzeigefläche (7') durch mindestens einen Anzeigeschirm einer Anzeigevorrichtung vom LCD-, Plasma- oder OLED-Typ und optional mit Touchscreen-Eigenschaften, gebildet ist.

5. Anordnung nach einem der vorangehenden Ansprüche, wobei die Bildsignal-Erzeugungsmittel (41 - 45) eine Bildelement-Speichereinheit (41) zum Speichern einer Vielzahl vordefinierter Bildelemente und wahlweise einen Bildelement-Mischer (43) zum Mischen von aus der Speichereinheit abgerufenen Bildelementen nach einer Vielzahl gespeicherter erster Mischprogramme aufweist.

6. Anordnung nach einem der vorangehenden Ansprüche, wobei die Bildsignal-Erzeugungsmittel (41 - 45) einen Videospeicher (42) zur Speicherung von Bildfolgen und wahlweise einen Video-Bildelement-Mischer (44) zum Mischen von aus dem Videospeicher ausgelesenen Bildfolgen, wahlweise mit aus der Bildelement-Speichereinheit (41) ausgelesenen Bildelementen, nach einer Vielzahl gespeicherter zweiter Mischprogramme aufweist.

7. Anordnung nach einem der vorangehenden Ansprüche, wobei die Anzeige-Ablaufsteuerung (46) einen Programmspeicher (47) zur Speicherung einer Vielzahl vorbestimmter Anzeige-Abläufe und/oder Geschwindigkeits- und/oder Relativpositions-Einstellmittel (48, 49) zur Geschwindigkeitseinstellung eines Anzeige-Ablaufes bzw. zur Vorgabe von Relativpositionen zwischen Bildelementen einer Anzeige aufweist.

8. Anordnung nach einem der vorangehenden Ansprüche, wobei die Anzeige-Ablaufsteuerung (46) einen Zufallsgenerator (45) zur zufalls-gesteuerten Bereitstellung von Bildsignalen, insbesondere zur zufalls-gesteuerten Zusammenstellung von aus der Bildelement-Speichereinheit (41) geladenen Bildelementen und/oder von aus dem Videospeicher (42) geladenen Bildfolgen, aufweist.

9. Anordnung nach einem der vorangehenden Ansprüche, mit einer Feedback-Einheit (54) zur Erfassung von Berührungen der Lauffläche durch den Probanden am Ort, an dem vorbestimmte Bildelemente angezeigt werden, oder an Orten, an denen kein vorbestimmtes Bildelement angezeigt wird, und zur Ausgabe eines Warn- oder Hinweissignals im Ansprechen hierauf.

10. Anordnung nach einem der vorangehenden Ansprüche, wobei unter der Lauffläche eine Anordnung von auf das Endlosband einwirkenden Aktoren (12) zur lokalen Verformung der Bandoberfläche zur Erzeugung von Unebenheiten und/oder lokaler Nachgiebigkeit bzw. Elastizität der Lauffläche vorgesehen oder das Laufband insgesamt auf Aktoren zur Erzeugung einer globalen Nachgiebigkeit bzw. Elastizität der gesamten Lauffläche gelagert ist.

11. Anordnung nach Anspruch 10, wobei der Anordnung von Aktoren (12) eine Profilsteuereinheit zugeordnet ist, die Steuersignale zur Betätigung der Aktoren zur Ausbildung eines vorbestimmten dynamischen Profils der Lauffläche ausgibt, und die Verarbeitungseinheit einen Steuersignalempfänger zum Empfang der durch die Profilsteuereinheit ausgegebenen Steuersignale als Positionszuordnungssignale aufweist und in der Verarbeitungseinheit ein Verarbeitungsalgorithmus implementiert ist, mit dem die Druckverteilungsbilder dem dynamischen Profil der Lauffläche zugeordnet werden.

12. Anordnung nach einem der vorangehenden Ansprüche, wobei ein vorderer Abschnitt der Lauffläche (2c; 2c'), wo die oder eine Anzeigefläche vorgesehen ist, in Gebrauchsstellung der Anordnung nach oben geneigt ist.

13. Anordnung nach einem der vorangehenden Ansprüche, mit einer Benutzerführungseinheit (54) zur optischen und/oder akustischen Ausgabe von anzeigebegleitenden Handlungsanweisungen, insbesondere über Ohrhörer und/oder Texteinblendungen im Bereich der Lauffläche.

14. Anordnung nach einem der vorangehenden Ansprüche, mit zusätzlichen optischen Anzeigemitteln zur Darstellung von Bildern auf einer weiteren Bildanzeigefläche, außerhalb der Lauffläche und insbesondere in Augenhöhe des Trainierenden, wobei die zusätzlichen Anzeigemittel ebenfalls mit den Bildsignal-Erzeugungsmitteln und der Anzeige-Ablaufsteuerung verbunden sind und insbesondere zu einer zusätzlichen Visualisierung der Lauffläche und/oder Laufumgebung genutzt werden.

## Claims

1. Arrangement (1) for training the gait, comprising:
an endless belt (2b) guided over at least two rollers (2a) and serving as a treadmill, the one surface of which serves as a walking surface (2c; 2c'),
a sensor system for determining a pressure/force distribution on a force measurement plate (3, 3') located underneath the walking surface, which is provided with a plurality of pressure/force sensors on the side facing the endless belt, an analyzing unit (4) connected, on the input side, to the pressure/force sensors, which detects pressure distributions generated by a walking or running subject on the walking surface,
a processing unit (51 - 53) connected, on the input side, to the analyzing unit, which generates from the pressure distributions values or signals, respectively, that characterize the gait of the subject,
optical display means (7; 7') for displaying images in the area of the walking surface, which are connected to image signal generating means (41 - 45) and a display process controller (46) for the generation and the position control in terms of time and space of images or image elements (49), and
a synchronization and adaptation stage (76) for synchronizing and adapting the processing of the pressure distribution images with/to the image representation in the area of the walking surface,
wherein the display means are provided with an image projector (7) for the projection of images onto the walking surface of the treadmill or
wherein the endless belt is formed of a transparent material at least in essential sections and an image display surface is provided underneath its, in the usage position, upper section.

2. Arrangement according to claim 1, wherein, in the usage position of the arrangement, the projector (7) is arranged in front of or in the front portion of and/or laterally above the same in such a way that the direction of projection encloses with the plane of the walking surface an acute angle.

3. Arrangement according to claim 2, wherein the projector (7) is received in a holder (7a), which is adjustable with respect to angles, to adjust a direction of projection, and an image signal distortion correcter (7b) is connected upstream thereof, which effects a distortion correction of images or image elements supplied by the image signal generating means in dependence on the adjusted angle between the direction of projection and the walking surface.

4. Arrangement according to one of the preceding claims, wherein the image display surface (7') is formed by at least one display screen of an electro-optical display device, particularly of the LCD, plasma or OLED type, and optionally with touchscreen properties.

5. Arrangement according to one of the preceding claims, wherein the image signal generating means (41 - 45) comprise an image element storing unit (41) for storing a plurality of predefined image elements and, optionally, an image element mixer (43) for mixing image elements retrieved from the storing unit according to a plurality of stored first mixing programs.

6. Arrangement according to one of the preceding claims, wherein the image signal generating means (41 - 45) comprise a video memory (42) for storing image sequences and, optionally, a video image element mixer (44) for mixing image sequences read out from the video memory optionally with image elements read out from the image element storing unit (41) according a plurality of stored second mixing programs.

7. Arrangement according to one of the preceding claims, wherein the display process controller (46) comprises a program memory (47) for storing a plurality of predetermined display processes and/or speed and/or relative position adjusting means (48, 49) for adjusting the speed of a display process and for predefining relative positions between image elements of a display.

8. Arrangement according to one of the preceding claims, wherein the display process controller (46) comprises a random generator (45) for the random-controlled provision of image signals, specifically for the random-controlled compilation of image elements loaded from the image element storing unit (41) and/or of image sequences loaded from the video memory.

9. Arrangement according to one of the preceding claims, comprising a feedback unit (51) for detecting contacts of the walking surface by the subject at the place where predetermined image elements are displayed, or at places where no predetermined image element is displayed, and for outputting a warning or information signal in response thereto.

10. Arrangement according to one of the preceding claims, wherein an array of actuators (12) acting on the endless belt for the local deformation of the belt surface in order to produce irregularities and/or a local resilience and elasticity of the walking surface is provided underneath the walking surface, or the treadmill as a whole is mounted on actuators for generating a global resilience and elasticity of the entire walking surface.

11. Arrangement according to claim 11, wherein the array of actuators (12) is assigned a profile control unit which outputs control signals for actuating the actuators in order to form a predetermined dynamic profile of the walking surface, and the processing unit comprises a control signal receiver for receiving the control signals, outputted by the profile control unit, as position assignment signals, and a processing algorithm is implemented in the processing unit by means of which the pressure distribution images are assigned to the dynamic profile of the walking surface.

12. Arrangement according to one of the preceding claims, wherein a front section of the walking surface (2c; 2c'), where the or a display surface is provided, is inclined in an upward direction in the usage position of the arrangement.

13. Arrangement according to one of the preceding claims, comprising a user guide unit (54) for the visual and/or audible output of display-accompanying instructions, particularly by means of earphones and/or text insertions in the area of the walking surface.

14. Arrangement according to one of the preceding claims, comprising additional optical display means for displaying images on another image display surface, outside of the walking surface and especially at eye level of the person doing the workout, wherein the additional display means are likewise connected to the image signal generating means and the display process controller, and are specifically used for an additional visualization of the walking surface and/or the walking environment.

## Revendications

1. Dispositif (1) pour l'entraînement à la marche, comprenant
une bande sans fin (2b), guidée via au moins deux galets de roulement (2a) et servant de tapis roulant, et dont une surface supérieure sert de surface de marche (2c ; 2c'),
un système capteur destiné à déterminer une distribution de pression/de force sur une plaque dynamométrique (3 ; 3') se trouvant sous la surface de marche et présentant, sur le côté dirigé vers la bande sans fin, une pluralité de capteurs de pression/de force,
une unité d'évaluation (4) reliée côté entrée aux capteurs de pression/de force, laquelle détecte des distributions de pression qui sont générées sur la surface de marche par un sujet qui marche ou qui court,
une unité de traitement (51 - 53) reliée côté entrée à l'unité d'évaluation, laquelle génère, à partir des distributions de pression, des valeurs ou signaux qui caractérisent la marche du sujet,
des moyens d'affichage optiques (7 ; 7') destinés à représenter des images dans la zone de la surface de marche, et qui sont reliés à des moyens générateurs de signaux d'image (41 - 45) et à une commande de déroulement d'affichage (4b) pour générer et commander en position spatiotemporelle (49) des images ou éléments d'image, ainsi que
un étage de synchronisation et d'adaptation (7b) destiné à synchroniser et adapter le traitement des images de distribution de pression avec la ou à la représentation d'image dans la zone de la surface de marche,
**caractérisé en ce que**
les moyens d'affichage présentent un projecteur d'images (7) pour la projection d'images sur la surface de marche du tapis roulant et la bande sans fin est formée au moins par sections principales de matériau transparent et une surface d'affichage d'images est prévue sous sa section supérieure en position d'usage, et la surface d'affichage d'images est formée par au moins un écran d'affichage d'un dispositif d'affichage électro-optique.

2. Dispositif selon la revendication 1, sachant que le projecteur (7) est disposé, en position d'usage du dispositif, devant ou dans la zone avant et/ou latéralement au-dessus de celui-ci de telle façon que la direction de projection forme un angle aigu avec le plan de la surface de marche.

3. Dispositif selon la revendication 2, sachant que le projecteur (7) est logé dans une fixation (7a) à réglage angulaire destinée à régler une direction de projection et un égaliseur de signaux d'image lui est antéposé, lequel provoque une égalisation d'images ou d'éléments d'image fournis par les moyens générateurs de signaux d'image en fonction de l'angle réglé entre la direction de projection et la surface de marche.

4. Dispositif selon l'une des revendications précédentes, sachant que la surface d'affichage d'image (7') est formée par au moins un écran d'affichage d'un dispositif d'affichage de type LCD, plasma ou OLED facultativement doté de propriétés d'écran tactile.

5. Dispositif selon l'une des revendications précédentes, sachant que les moyens générateurs de signaux d'image (41 - 45) présentent une unité de mémoire d'éléments d'image (41) destinée à mémoriser une pluralité d'éléments d'image prédéfinis et facultativement un mélangeur d'éléments d'image (43) destiné à mélanger des éléments d'image rappelés de l'unité de mémoire selon une pluralité de premiers programmes de mélange mémorisés.

6. Dispositif selon l'une des revendications précédentes, sachant que les moyens générateurs de signaux d'image (41 - 45) présentent une mémoire vidéo (42) destinée à mémoriser des séquences d'images et facultativement un mélangeur d'éléments d'images vidéo (44) destiné à mélanger des séquences d'images extraites de la mémoire vidéo, facultativement avec des éléments d'image extraits de l'unité de mémoire d'éléments d'image (41), selon une pluralité de deuxièmes programmes de mélange mémorisés.

7. Dispositif selon l'une des revendications précédentes, sachant que la commande de déroulement d'affichage (46) présente une mémoire de programmes (47) destinée à mémoriser une pluralité de déroulements d'affichage prédéterminés et/ou des moyens de réglage de vitesse et/ou de position relative (48, 49) destinés à régler la vitesse d'un déroulement d'affichage ou à spécifier des positions relatives entre des éléments d'image d'un affichage.

8. Dispositif selon l'une des revendications précédentes, sachant que la commande de déroulement d'affichage (46) présente un générateur aléatoire (45) destiné à mettre à disposition par commande aléatoire des signaux d'image, en particulier à assembler par commande aléatoire des éléments d'image chargés à partir de l'unité de mémoire d'éléments d'image (41) et/ou des séquences d'images chargées à partir de la mémoire vidéo (42).

9. Dispositif selon l'une des revendications précédentes, comprenant une unité de rétroaction (54) destinée à saisir des contacts de la surface de marche par le sujet à l'emplacement auquel des éléments d'image prédéterminés sont affichés, ou à des emplacements auxquels aucun élément d'image prédéterminé n'est affiché, et à émettre en réponse à cela un signal d'avertissement ou d'indication.

10. Dispositif selon l'une des revendications précédentes, sachant que sous la surface de marche, un dispositif d'actionneurs (12) intervenant sur la bande sans fin, destiné à déformer localement la surface supérieure de bande, est prévu pour générer des inégalités et/ou une souplesse ou élasticité locale de la surface de marche ou le tapis roulant est entièrement logé sur des actionneurs pour générer une souplesse ou élasticité globale de toute la surface de marche.

11. Dispositif selon la revendication 10,
sachant qu'au dispositif d'actionneurs (12) est attribuée une unité de commande de profil qui émet des signaux de commande destinés à actionner les actionneurs afin de constituer un profil dynamique prédéterminé de la surface de marche, et l'unité de traitement présente un récepteur de signaux de commande destiné à recevoir les signaux de commande émis par l'unité de commande de profil en tant que signaux d'attribution de position, et un algorithme de traitement avec lequel les images de distribution de pression peuvent être attribuées au profil dynamique de la surface de marche est implémenté dans l'unité de traitement.

12. Dispositif selon l'une des revendications précédentes, sachant qu'une section avant de la surface de marche (2c ; 2c') où la ou une surface d'affichage est prévue est inclinée vers le haut en position d'usage du dispositif.

13. Dispositif selon l'une des revendications précédentes, comprenant une unité de guidage d'utilisateur (54) destinée à émettre optiquement et/ou acoustiquement des instructions de manipulation qui accompagnent l'affichage, en particulier via des écouteurs et/ou des incrustations de texte dans la zone de la surface de marche.

14. Dispositif selon l'une des revendications précédentes, comprenant des moyens d'affichage optiques supplémentaires destinés à représenter des images sur une surface d'affichage d'images supplémentaire, en dehors de la surface de marche et en particulier à hauteur des yeux de la personne qui s'entraîne, sachant que les moyens d'affichage supplémentaires sont également reliés aux moyens générateurs de signaux d'image et à la commande de déroulement d'affichage et sont en particulier utilisés pour une visualisation supplémentaire de la surface de marche et/ou de l'environnement de marche.
